# FASCICULE DE BREVET EUROPEEN

(11) **EP 0 581 686 B1**
(45) Date de publication et mention de la délivrance du brevet: **29.10.1997**
(21) Numéro de dépôt: 93401981.1
(22) Date de dépôt: 30.07.1993
(51) Int. Cl.: G01V 3/22

(54) **Procédé et dispositif de sondage et de contrôle d'un volume de sous-sol**
Verfahren und Vorrichtung zum Abtasten und zum Untersuchen eines Untergrundvolumens
Method and apparatus for probing and inspecting an underground volume

(30) Priorité: 31.07.1992 FR 9209515; 28.12.1992 FR 9215759
(43) Date de publication de la demande: 02.02.1994
(60) Demande divisionnaire: 97106066.0
(73) Titulaire: EUGESOL, F-92000 Nanterre (FR)
(72) Inventeur: Lantier, François, F-38290 Villefontaine (FR); Frappin, Pierre, F-38290 Villefontaine (FR)
(74) Mandataire: Dronne, Guy

(56) Documents cités:
- FR-A- 1 247 925
- US-A- 2 654 064
- US-A- 2 779 912
- US-A- 3 134 941

## Description

La présente invention a pour objet un procédé de d'investigation d'un volume de sous-sol destiné à permettre, par une opération simple, la connaissance de la nature et de la consistance des terrains.

Le problème de la nature du sous-sol se pose pour la construction d'ouvrages importants, le percement de tunnels et la recherche pétrolière, entre autres.

Dans le domaine de la recherche pétrolière, il est connu, (depuis 1927, C. SCHLUMBERGER) de réaliser des diagraphies différées et, au moyen de celles-ci, d'obtenir la mesure de résistivité des formations traversées le long d'un sondage préalable à un forage. A cet effet, on utilise une sonde dite normale portant de quatre à six électrodes à l'aide desquelles est mesuré le log de résistivité.

Le principe de cette mesure consiste à injecter un courant continu dans le terrain, sous une tension de 200 à 600 volts avec une intensité de 200 à 400 milliampères, à mesurer la différence de potentiel induite entre deux électrodes de mesure, et à en déduire la résistivité locale, le résultat étant disponible en Ohms-mètres. Mais, avec un tel système, l'investigation latérale est de l'ordre d'un mètre autour du sondage. Il en résulte que, pour connaître la nature exacte du sous-sol, les forages doivent être multipliés ce qui est long et, par suite, coûteux.

US-A-2 654 064 décrit un dispositif de sondage du milieu adjacent à un puits. Ce sondage a pour but de déterminer l'extension de l'invasion de la boue dans le puits par une mesure de conductivité électrique. A cet effet plusieurs électrodes de potentiel A1-A11 sont alimentées successivement et l'électrode M donne des mesures correspondantes de résistivité.

FR-B- 1 247 925 décrit un dispositif de sondage dans lequel trois électrodes d'émission de courant et quatre électrodes de mesure permettent d'obtenir trois mesures de la résistivité des terrains autour du forage. Mais ces informations, permettant d'établir un profilage, sont insuffisantes pour détecter la présence, à une distance supérieure à un mètre du forage, d'une hétérogénéité lithologique.

Dans les deux cas, les mesures effectuées sont des mesures relatives aux couches entourant immédiatement le puits et la sonde se déplace à l'intérieur de celui-ci.

Or, ce qui importe dans certaines applications, notamment dans les travaux publics est, non seulement la connaissance des couches traversées, mais également la connaissance de l'environnement du forage.

La présente invention a pour objet un procédé d'investigation d'un volume de sous-sol permettant de pallier cet inconvénient et de permettre en une seule opération la détermination de l'homogénéité de nature d'un volume de sous-sol important, permettant d'établir une cartographie du sous-sol. A titre d'exemple, la présente invention permet d'ausculter un volume cylindrique d'un diamètre de 1 à 100 mètres autour d'un trou de sondage que ce trou soit vertical, horizontal ou incliné.

Un second objet de la présente invention est de permettre un contrôle de la qualité de l'injection effectuée de manière à répondre aux normes de sécurité.

Selon la présente invention, le procédé d'investigation d'un volume de sous-sol par mesure électrique de la résistivité du sol, un courant continu étant appliqué par plusieurs électrodes (A) et une électrode (B) écartées l'une de l'autre, la tension développée étant mesurée entre deux électrodes de potentiel (M), est caractérisé en ce qu'il consiste à disposer dans un forage une sonde fixe portant un ensemble d'électrodes (A) d'application d'une tension électrique et un ensemble d'électrodes (M) de mesure de potentiel, le nombre d'électrodes (A) étant supérieur à celui des électrodes (M), les électrodes (A) étant régulièrement réparties, les électrodes (M) étant intercalées entre les électrodes centrales (A) dans des positions fixes, l'électrode (B) étant repoussée à l'infini, les électrodes (A) étant alimentées successivement.

La sonde selon l'invention utilisant la technique pôle-dipôle, met en oeuvre, par exemple, trente cinq électrodes disposées sur toute la longueur ou hauteur du forage (quinze, trente ou soixante mètres). La seconde électrode d'émission de courant B est reportée à l'infini, (pratiquement quelques centaines de mètres) et l'électrode A est "mobile", les électrodes A étant successivement alimentées. Le nombre d'électrodes A est supérieur au nombre d'électrodes M de manière à pouvoir faire varier la distance A-Ma,Mb pour obtenir un sondage large autour de la sonde, Ma,Mb désignant un couple quelconque d'électrodes de mesure. En injectant un courant continu sur les électrodes A, on obtient en appliquant la loi d'Ohm généralisée à un tripôle ( A, Ma-Mb ) une mesure de résistivité apparente des terrains entre les deux électrodes de potentiel Ma-Mb fixes. Plus l'électrode A s'éloigne de Ma et Mb, plus la résistivité apparente affecte une épaisseur de terrains plus grande à partir de l'axe A,Ma, Mb c'est à dire à partir de la sonde. Ce résultat résulte de la forme des lignes équipotentielles qui se développent. L'électrode active d'émission apparaît en quelque sorte mobile le long de la sonde ce qui permet d'obtenir des volumes d'exploration importants étant donné que c'est la distance électrode d'injection- électrodes de potentiel qui détermine le profondeur des équipotentielles. Cette profondeur peut ainsi être importante de même que le nombre de points de mesure.

Selon une autre caractéristique de l'invention, le procédé de contrôle de la répartition d'une injection de béton dans le sol est caractérisé en ce qu' il consiste, avant injection à disposer, dans une cavité ou au sol, une pluralité de sondes parallèles chacune portant un ensemble d'électrodes d'émission (A) et un ensemble d'électrodes de mesure (M) réparties selon un pas fixe sur toute la longueur de la sonde et à alimenter successivement toutes les électrodes (A), puis après coulée du béton à recommencer la même opération avec la même disposition d'électrodes pour déterminer, par différence, la répartition du béton dans un volume donné.

Elle est basée sur le fait que le béton à l'état liquide ou pâteux est un bon conducteur de l'électricité immédiatement après son injection alors qu'il devient très résistant après durcissement. On détecte les variations qui se sont produites entre l'état "0", avant injection et l'état "1" après injection pour contrôler le succès ou l'échec de l'injection.

D'autres caractéristiques et avantages de l'invention apparaîtront au cours de la description qui va suivre d'un mode de réalisation particulier, donné à titre d'exemple non limitatif en regard des dessins qui représentent :
- La Figure 1, un exemple de positionnement des électrodes d'injection et de potentiel;
- La Figure 2, un tableau des niveaux de mesures;
- La figure 3 un exemple de réalisation d'une sonde :
- La Figure 4, deux exemples de résultat de mesures relevées dans un terrain particulier;
- La Figure 5, un exemple du résultat obtenu par un contrôle selon l'invention

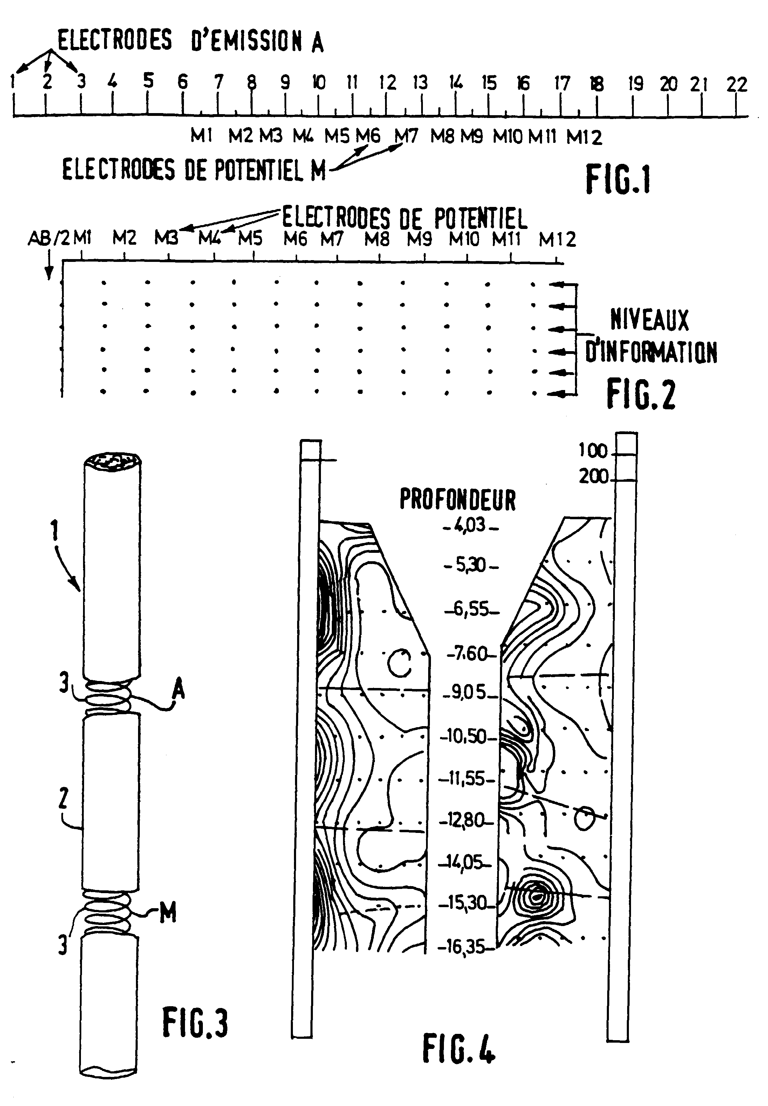

Sur la figure 1, on voit que l'électrode d'injection A peut prendre un grand nombre de positions, vingt trois dans l'exemple représenté. Il s'agit en fait de vingt trois électrodes qui sont successivement alimentées par un générateur émettant un courant continu de 10 à 300 mA sous une tension de 200 volts, par exemple.

Le générateur est inclus dans un coffret comprenant un multiplexeur manuel et un milliampèremètre digital. le dispositif comprend également un coffret de réception incluant un multiplexeur automatique et un millivoltmètre digital. Une centrale d'acquisition et de gestion comprend un ordinateur PC 386 avec son programme de gestion des mesures et des moyens de mémorisation.

Comme schématisé sur la figure 1, les électrodes de potentiel M sont au nombre de douze et occupent les positions M1 à M12. Ces positions sont intercalées avec les vingt trois positions de l'électrode d'injection A. En faisant varier les positions de A et des couples Ma-Mb , on obtient, comme représenté sur la figure 2, cent trente deux mesures de résistivité avec six niveaux d'information disposés au droit des centres de symétrie de chaque couples Ma-Mb en fonction de la demi longueur AB et donc en fonction de la profondeur. Au droit de chaque couple Ma-Mb, on obtient deux mesures de résistivité par les deux positions symétriques de A par rapport à Ma-Mb.

L'écartement de deux électrodes M peut être quelconque, mais fixe pour un ensemble de mesures données. Les positions relatives de A sont alors parfaitement déterminées comme représenté sur la figure 1. Les électrodes sont incluses dans un câble et, pour assurer un bon contact des électrodes et du terrain, on remplit, avant les mesures, le trou de forage avec de l'eau ou de la boue.

Toute anomalie de résistivité est repérée et projetée, pour les résultats sur un rayon ainsi que son niveau. Or, les variations de résistivité indiquent des variations de structure du sous-sol. La résistivité est un bon indice de la qualité des terrains en matière de travaux souterrains puisque la plupart des accidents géologiques générateurs de difficultés lors de l'excavation et du soutènement sont argileux avec éventuellement présence d'eau. Ils se traduisent généralement par des résistivités faibles.

A contrario, les calcaires ont une résistivité plus élevée qui croît avec les roches primaires telles que le granit, par exemple. Le cylindre permet ainsi de déceler, dans un volume rocheux donné, autour d'un forage toute zone anormale dans un environnement à priori homogène et de la situer précisément en position par rapport aux électrodes de potentiel M. Mais, l'azimut autour du forage n'est bien entendu pas maîtrisé. on obtient ainsi une projection plane du cylindre électrique.

La figure 3 représente une partie d'une sonde selon l'invention. Elle se compose d'un câble isolé 1 comprenant, par exemple, quarante huit conducteurs qui sont successivement dégagés de la gaine 2 pour former une boucle 3 qui est ainsi en contact avec le terrain. L'alimentation des boucles A d'injection et des boucles M sont gérées respectivement par les multiplexeurs mentionnés précédemment. Bien entendu, le nombre d'électrodes d'émission et de mesure peut être quelconque, le nombre d'électrodes démission étant toujours supérieur au nombre d'électrodes de mesure.

La figure 4 représente le résultat obtenu au cours de deux forages de prospection. Ces forages peuvent être de faible section puisque la section de la sonde est elle-même très faible. Les deux schémas représentent des pseudo-sections qui rendent compte de toute anomalie détectée dans un cylindre entourant la sonde. Les lignes figurant à l'intérieur de chaque section sont des lignes d'isorésistivité, cette grandeur étant exprimée en Ohms.mètres

En haut de la ligne représentant le forage, on trouve de la terre végétale sur une hauteur de l'ordre de 1 mètre, puis, dans l'exemple représenté, des couches de calcaire gris bleuté légèrement argileux. Sur la figure de gauche, on voit que les lignes d'isorésistivité sont concentrées autour du trou de forage. Il s'agit, et cela a été corroboré par un carottage, d'une couche dense de calcaire massif gris bleuté légèrement argileux. Mais dès que l'on s'éloigne de l'axe du forage, les lignes deviennent plus espacées ce qui traduit une résistivité plus faible. Il s'agit de couches d'argile plus ou moins déconsolidé avec quelques passages de blocs de calcaire. La figure de droite représente les résultats d'un forage qui a été effectué au voisinage du premier forage et qui confirme l'existence de couches d'argile.

Le procédé selon l'invention rend compte de tout accident géologique se produisant dans un rayon donné autour du forage de sondage, mais sans indiquer la position exacte de cet accident.

Les applications du procédé sont immédiates: Reconnaissance autour de forages verticaux. L'investigation latérale est fonction de la profondeur réelle de l'ouvrage et peut être augmentée; reconnaissance à l'avancement dans des forages horizontaux, notamment dans le cas d'ouvrages souterrains avec tunnelier et elle permet de repérer les karsts ou dolines ainsi que les hétérogénéités lithologiques. Il peut également être mis en oeuvre dans les forages pétroliers pour repérer les structures et dans les opérations préalables à la fracturation hydraulique des roches.

La présente invention vise également un procédé électrique et un dispositif de contrôle d'injections de béton dans le sol. Dans une telle application, la nature du sous-sol au voisinage de l'ouvrage est connue et c'est pour remédier à des faiblesses détectées que l'on injecte du béton.

De telles injections sont courantes quand il s'agit de réaliser une consolidation dans le sol en vue d'implanter un ouvrage d'art tel qu'un tunnel lorsque le sol présente une structure poreuse et insuffisamment résistante. Dans ces conditions, on fore un trou et l'on injecte un coulis de béton qui se répand dans le sol. Elles sont aussi réalisées, lorsqu'il s'agit d'effectuer une consolidation, autour de galeries et tunnels, au sein de massifs rocheux, dans des formations meubles (par exemple du sable) , dans des remblais ou dans les carrières. Malheureusement, compte tenu de l'hétérogénéité des sols, le coulis peut se diriger dans des zones non désirées et laisser creuses les zones intéressant l'implantation de l'ouvrage. Il est également souhaitable de vérifier l'injection réalisée dans les voiles de béton utilisés pour les barrages et dans les têtes de tunnel.

A partir d'un état "0", après injection d'un coulis de béton dans le sol, on procède à une seconde série de mesures de l'état "1". Ces mesures sont effectuées moins de trois semaines après l'injection du coulis pour que celui-ci soit encore bon conducteur électrique, sa conductibilité s'atténuant au fur et à mesure de sa prise.

Des mesures strictement identiques à la mesure effectuée dans l'état "0" sont alors réalisées avec le même nombre de sondes, la même disposition géométrique et la même chaîne de mesure.

On compare ensuite les six niveaux d'information état "1" aux six niveaux état "0" le rapport obtenu mesurant les variations de résistivité. On obtient ainsi par contraste une représentation graphique des variations de résistivité entre les deux états. Les valeurs de résistivité mesurées dans l'état "1" sont en général plus faibles que celles mesurées dans l'état "0" car les coulis sont en général de 5 à 50 fois plus conducteurs que les terrains.

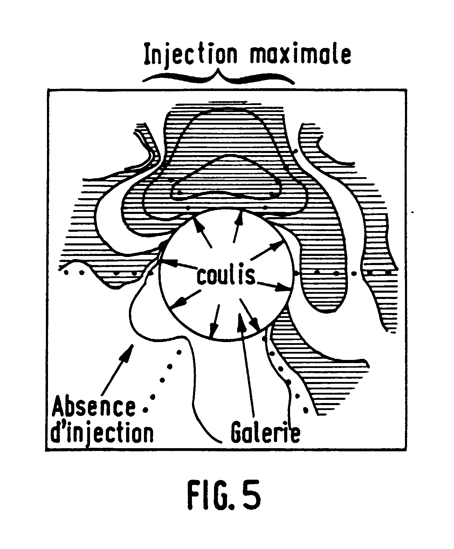

La figure 5 montre la répartition d'un coulis derrière le parement d'une galerie avec en radier une absence d'injection , se traduisant par un rapport 1 entre les deux états.

## Revendications

1. Procédé d'investigation d'un volume de sous-sol par mesure électrique de la résistivité du sol, un courant continu étant appliqué par plusieurs électrodes (A) et d'une électrode (B) écartées l'une de l'autre, la tension développée étant mesurée entre deux électrodes de potentiel (M), caractérisé en ce qu'il consiste à disposer dans un forage une sonde fixe portant un ensemble d'électrodes (A) d'application d'une tension électrique et un ensemble d'électrodes (M) de mesure de potentiel, le nombre d'électrodes (A) étant supérieur à celui des électrodes (M), les électrodes (A) étant régulièrement réparties, les électrodes (M) étant intercalées entre les électrodes centrales (A) dans des positions fixes, l'électrode (B) étant repoussée à l'infini, les électrodes (A) étant alimentées successivement par un multiplexeur d'émission relié aux électrodes (A), un multiplexeur de lecture relié aux électrodes (M), étant connecté à une centrale d'acquisition et de mesure.

2. Procédé de contrôle de la répartition d'une injection de béton dans le sol par mise en oeuvre du procédé selon la revendication 1, caractérisé en ce qu'il consiste :
- avant injection à disposer, dans une cavité ou au sol, une pluralité de sondes parallèles, chacune portant un ensemble d'électrodes d'injection (A) et un ensemble d'électrodes de mesure (M) réparties selon un pas fixe sur toute la longueur de la sonde et à alimenter successivement toutes les électrodes (A);
- puis après coulée du béton à recommencer la série de mesures pour déterminer, par différence, la répartition du béton dans un volume donné.

3. Sonde pour la mise en oeuvre du procédé selon la revendication 1, caractérisée en ce qu'elle comprend un câble isolé (1) renfermant à l'intérieur d'une gaine (2) des conducteurs multiples qui sont successivement dégagés pour constituer des boucles (3) en contact avec le terrain.

## Patentansprüche

1. Verfahren zur Untersuchung eines Untergrundvolumens durch elektrische Messung des Bodenwiderstands, wobei ein Gleichstrom mittels mehrerer Elektroden (A) und einer Elektrode (B), die voneinander entfernt sind, angelegt wird und wobei die ausgebildete Spannung zwischen zwei Potentialelektroden (M) gemessen wird, dadurch gekennzeichnet, daß es darin besteht, in einer Bohrung eine fixe Sonde anzuordnen, die einen Satz von Elektroden (A) zum Anlegen einer elektrischen Spannung und einen Satz von Elektroden (M) zur Messung des Potentials trägt, wobei die Anzahl der Elektroden (A) größer ist als die der Elektroden (M), die Elektroden (A) regelmäßig verteilt sind, die Elektroden (M) zwischen den zentralen Elektroden (A) an fixen Positionen eingefügt sind, die Elektrode (B) ins Unendliche verschoben ist, die Elektroden (A) nacheinander durch einen mit den Elektroden (A) verbundenen Emissionsmultiplexer gespeist werden und wobei ein Lesemultiplexer mit den Elektroden (M) verbunden ist, der mit einer Aufnahme- und Meßzentrale verbunden ist.

2. Verfahren zur Überwachung der Verteilung einer Betoninjektion in den Boden durch Anwendung des Verfahrens gemäß Anspruch 1, dadurch gekennzeichnet, daß es besteht aus:
- dem Anordnen einer Mehrzahl paralleler Sonden, vor der Injektion, in einer Vertiefung oder am Boden, wobei jede einen Satz von Injektionselektroden (A) und einen Satz von Meßelektroden (M) trägt, die gemäß einem fixen Weg über die gesamte Länge der Sonde verteilt sind, und der sukzessiven Speisung aller Elektroden (A);
- dann, nach dem Gießen des Betons, dem Wiederbeginn einer Meßserie, um, durch Differenz, die Verteilung des Betons in einem gegebenen Volumen zu bestimmen.

3. Sonde zur Durchführung des Verfahrens gemäß Anspruch 1, dadurch gekennzeichnet, daß sie ein isoliertes Kabel (1) umfaßt, das im Inneren eines Mantels (2) zahlreiche Leiter umfaßt, die nacheinander freigelegt sind, um Windungen (3) in Kontakt mit dem Boden zu bilden.

## Claims

1. Method for investigating a volume of subsoil by electrical measurement of the resistivity of the soil, a direct current being applied by several electrodes (A) and from one electrode (B) separated from one another, the voltage developed being measured between two potential electrodes (M), characterised in that it consists in arranging in a borehole a fixed probe carrying a set of electrodes (A) for applying an electric voltage and a set of electrodes (M) for measuring potential, the number of electrodes (A) being greater than that of the electrodes (M), the electrodes (A) being distributed at regular intervals, the electrodes (M) being interpolated between the central electrodes (A) in fixed positions, the electrode (B) being pushed on towards infinity, the electrodes (A) being supplied with power successively by a transmitting multiplexer connected to the electrodes (A), a reading multiplexer connected to the electrodes (M) being connected to a central capture and measurement unit.

2. Method for checking the distribution of an injection of concrete into the soil by implementing the method according to Claim 1, characterised in that it consists:
- before injection, in arranging, in a cavity or on the soil, a plurality of parallel probes, each carrying a set of injection electrodes (A) and a set of measurement electrodes (M) distributed at a fixed pitch over the whole length of the probe and in supplying power to each of the electrodes (A) in succession;
- then, after casting the concrete, in starting the series of measurements again in order to determine, by finding the difference, the distribution of the concrete in a given volume.

3. Probe for implementing the method according to Claim 1, characterised in that it comprises an insulated cable (I) enclosing within a sheath (2) multiple conductors, which are cleared successively in order to constitute loops (3) in contact with the earth.
